(19) Europäisches Patentamt
European Patent Office
Office européen des brevets

(11) **EP 3 355 057 A2**

(12) **EUROPEAN PATENT APPLICATION**

(43) Date of publication:
**01.08.2018 Bulletin 2018/31**

(51) Int Cl.:
***G01N 33/574*** (2006.01)

(21) Application number: **17209422.9**

(22) Date of filing: **26.05.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2010 US 350042 P**
**01.06.2010 EP 10164624**

(62) Document number(s) of the earlier application(s) in accordance with Art. 76 EPC:
**11724588.6 / 2 577 314**

(71) Applicants:
- **metanomics Health GmbH**
  **10589 Berlin (DE)**
- **Universitätsklinikum Schleswig-Holstein**
  **24105 Kiel (DE)**
- **Ernst-Moritz-Arndt-Universität Greifswald**
  **17487 Greifswald (DE)**

(72) Inventors:
- **Kamlage, Beate**
  **12161 Berlin (DE)**

- **Reszka, Regina**
  **16341 Panketal (DE)**
- **Kluttig, Martin**
  **67433 Neustadt (DE)**
- **Kalthoff, Holger**
  **24106 Kiel (DE)**
- **Schniewind, Bodo**
  **24105 Kiel (DE)**
- **Mayerle, Julia**
  **80469 München (DE)**
- **Lerch, Markus**
  **17489 Greifswald (DE)**

(74) Representative: **Herzog, Fiesser & Partner**
**Patentanwälte PartG mbB**
**Dudenstrasse 46**
**68167 Mannheim (DE)**

Remarks:
This application was filed on 21-12-2017 as a divisional application to the application mentioned under INID code 62.

(54) **MEANS AND METHODS FOR DIAGNOSING PANCREATIC CANCER IN A SUBJECT**

(57) The present invention relates to the field of diagnostic methods. Specifically, the present invention contemplates a method for diagnosing pancreatic cancer in a subject, a method for identifying whether a subject is in need for a therapy of pancreatic cancer or a method for determining whether a pancreatic cancer therapy is successful. The invention also relates to tools for carrying out the aforementioned methods, such as diagnostic devices.

**EP 3 355 057 A2**

**Description**

The present invention relates to the field of diagnostic methods. Specifically, the present invention contemplates a method for diagnosing pancreatic cancer in a subject, a method for identifying whether a subject is in need for a therapy of pancreatic cancer or a method for determining whether a pancreatic cancer therapy is successful. The invention also relates to tools for carrying out the aforementioned methods, such as diagnostic devices.

Pancreatic cancer has the worst prognosis of all solid tumors with 5-year survival rates of less than 5% but an increasing incidence (Everhart 2009, Gastroenterology 136:1134-11449). There is a widely acknowledged demand for the establishment of innovative tools and technologies for point-of-care utilization of specific biomarkers and novel molecular imaging tools for early diagnosis, prognostic stratification and differential diagnosis of pancreatic cancer. Advances in these areas are pivotal to improve the prognosis of this malignancy, since timely surgical resection of early stage tumors is currently the only effective means of treatment of this dismal disease.

The mortality of this cancer type is the highest of any cancer type in Europe and the western world. People die soon after diagnosis due to the lack of means for early detection. Early symptoms are rare and uncharacteristic. Thus, PDACs are commonly diagnosed in an advanced stage of the disease. To date, the best imaging technologies to detect PDAC are endoscopic ultrasound (EUS), spiral computer tomography (CT), magnetic resonance cholangiopancreatography (MRCP) or endoscopic retrograde cholangiopancreatography (ERCP) (Dewitt 2006, Gastroenterol Hepatol. (4):717-25). Unfortunately, the resolution of these technologies for detecting neoplastic lesions within the pancreas is in the range of 3-10 mm. Thus, they are not able to detect pancreatic neoplasia at a curable stage. The serum concentration of conventional tumor markers such as CA19-9 is increased in a subset of pancreatic cancer patients (Fry 2008, Langenbecks Arch Surg. (393): 883-90). However, so far all available markers lack sensitivity and tumor specificity. Thus, new approaches are urgently needed to increase the diagnostic sensitivity towards the detection of very small, early stage PDAC and its precursor lesions (PanINs and IPMNs) as well as prognostic subgroups of advanced tumors.

The association between chronic inflammation and the development of malignancies has been recognized for many years. For pancreatic cancer this association was only recently confirmed and a consensus conference agreed upon a new classification for pancreatic intraepithelial neoplasia as noninvasive precursor lesions (Hruban 2004, Am J Surg Path (28): 977-987). Chronic pancreatitis is defined as recurrent bouts of a sterile inflammatory disease characterized by often progressive and irreversible morphological changes, typically causing pain and permanent impairment of pancreatic function. With an incidence of 8.2, a prevalence of 27.4 per 100 000 population and a 0.04% to 5% frequency in unselected autopsy specimens chronic pancreatitis represents a frequent disorder of the gastrointestinal tract. Various etiologies are responsible for the development of chronic pancreatitis. An increased risk of patients suffering from of chronic pancreatitis to die from pancreatic cancer was shown in an international cooperative investigation conducted by AB Lowenfels and coworkers as a multicenter historical cohort study of 2015 patients with chronic pancreatitis recruited from clinical centers in 6 countries in 1993. This study found a cumulative risk of pancreatic cancer in patients with chronic pancreatitis of 1.8% after 10 years and of 4% after 20 years with a standardized incidence ratio of 14.4. For patients with a minimum of two years follow up the risk of pancreatic cancer was 16.5 fold higher than that of the general population (Lowenfels 1993, N Engl J Med (328): 1433-1437). The search for an association between chronic pancreatitis and pancreatic cancer intensified when in 1996 a single point mutation in the third exon of the cationic trypsinogen gene on chromosome 7 (7q35) was found to be associated with hereditary pancreatitis and multiple kindreds were subsequently identified and reported. Only very recently the EUROPAC study group presented their work on clinical and genetic characteristics in hereditary pancreatitis. In a multilevel proportional hazard model employing data obtained from the European Registry of Hereditary Pancreatitis this group presented 112 families in 14 countries (418 affected individuals) (Howes 2004, Clinical Gastroenterology and Hepatology (2): 252-261). The cumulative risk (95% CI) of pancreatic cancer was 44.0% (8.0% - 80.0%) at 70 years from symptom onset with a standardized incidence ratio of 67% (50% - 82%). A previous study had also shown an estimated lifetime risk of pancreatic cancer of 40% (Lowenfels 2001, JAMA 286: 169-170, Lowenfels 1997, J Natl Cancer Inst 89: 442-44656).

In pancreatic cancer imaging studies fail to detect early pancreatic malignancies in a curable stage, however in the background of chronic pancreatitis imaging studies such as EUS, CT or MRI drop sensitivity and specificity to a degree where tossing a coin is equally reliable. Serum markers would therefore be an irreplaceable tool to detect pancreatic malignancy in a high risk cohort.

There are a few reports of metabolic changes in patients suffering pancreas-associated diseases. Schrader et al (Schrader 2009, Panceas 38: 416-421) suggests that patients with pancreatic cancer and chronic pancreatitis show significant changes in serum aminoacid levels. It has been suggested that among the ceramides sphingomyelin on the cell surface of cancer cells takes actively part in cell signalling. Ceramides are known to induce apoptosis in cancer cells. Low levels of sphingomyelin suggest less responsiveness to gemcitabine treatment (Modrak 2009, Mol Cancer Res 7:890-896).

In conclusion with a 5-year survival rate of 0.5-5%, pancreatic cancer carries the most dismal prognosis of all human tumors and represents the 4th leading cause in cancer-related deaths worldwide. It is thus a disease with a major socioeconomic impact. Accurate diagnosis and timely surgical resection of early tumors currently offer the only realistic prospect for the improvement of patient prognosis.

Thus, the present invention relates to a method for diagnosing pancreas cancer in a subject comprising the steps of:

(a) determining in a sample of a subject suspected to suffer from pancreas cancer the amount of at least one biomarker from Tables 2a, 2b, 3a, 3b; and
(b) comparing the said amount of the at least one biomarker with a reference, whereby pancreas cancer is to be diagnosed.

The method as referred to in accordance with the present invention includes a method which essentially consists of the aforementioned steps or a method which includes further steps. However, it is to be understood that the method, in a preferred embodiment, is a method carried out ex vivo, i.e. not practised on the human or animal body. The method, preferably, can be assisted by automation.

The term "diagnosing" as used herein refers to assessing whether a subject suffers from the pancreatic cancer, or not. As will be understood by those skilled in the art, such an assessment, although preferred to be, may usually not be correct for 100% of the investigated subjects. The term, however, requires that a statistically significant portion of subjects can be correctly assessed and, thus, diagnosed. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 50%, at least 60%, at least 70%, at least 80%, at least 90% or at least 95%. The p-values are, preferably, 0.2, 0.1, or 0.05.

The term includes individual diagnosis of pancreatic cancer or its symptoms as well as continuous monitoring of a patient. Monitoring, i.e. diagnosing the presence or absence of pancreatic cancer or the symptoms accompanying it at various time points, includes monitoring of patients known to suffer from pancreatic cancer as well as monitoring of subjects known to be at risk of developing pancreatic cancer. Furthermore, monitoring can also be used to determine whether a patient is treated successfully or whether at least symptoms of pancreatic cancer can be ameliorated over time by a certain therapy.

The term "pancreatic cancer" or "pancreas cancer" as used herein relates to cancer which is derived from pancreatic cells. Preferably, pancreatic cancer as used herein is pancreatic adenocarcinoma. The symptoms accompanying pancreatic cancer are well known from standard text books of medicine such as Stedmen or Pschyrembl.

The term "biomarker" as used herein refers to a molecular species which serves as an indicator for a disease or effect as referred to in this specification. Said molecular species can be a metabolite itself which is found in a sample of a subject. Moreover, the biomarker may also be a molecular species which is derived from said metabolite. In such a case, the actual metabolite will be chemically modified in the sample or during the determination process and, as a result of said modification, a chemically different molecular species, i.e. the analyte, will be the determined molecular species. It is to be understood that in such a case, the analyte represents the actual metabolite and has the same potential as an indicator for the respective medical condition.

Moreover, a biomarker according to the present invention is not necessarily corresponding to one molecular species. Rather, the biomarker may comprise stereoisomers or enantiomeres of a compound. Further, a biomarker can also represent the sum of isomers of a biological class of isomeric molecules. Said isomers shall exhibit identical analytical characteristics in some cases and are, therefore, not distinguishable by various analytical methods including those applied in the accompanying Examples described below. However, the isomers will share at least identical sum formula parameters and, thus, in the case of, e.g., lipids an identical chain length and identical numbers of double bonds in the fatty acid and/or sphingo base moieties

In the method according to the present invention, at least one metabolite of the biomarkers shown in Tables 2a, 2b, 3a, 3b is to be determined. However, more preferably, a group of biomarkers will be determined in order to strengthen specificity and/or sensitivity of the assessment. Such a group, preferably, comprises at least 2, at least 3, at least 4, at least 5, at least 10 or up to all of the said biomarkers shown in the Tables 2a, 2b, 3a, 3b.

Preferably, the at least one biomarker determined in the method of the present invention is a biomarker of category 1 as shown in Table 2a and 2b or 2 as shown in Table 3a and 3b. More preferably, the at least one biomarker is a biomarker of category 1 or 2 and, most preferably, it is a biomarker of category 1.

More preferably, the at least one biomarker determined in the method of the present invention is an amino acid as shown in Table 2a, most preferably, Proline, Threonine, Ornithine or trans-4-hydroxyproline. If more than one biomarker is to be determined, it is preferably envisaged that the said more biomarkers encompass Proline, Threonine, Ornithine and/or trans-4-hydroxyproline and, preferably, all of the said amino acids.

More preferably, the at least one biomarker determined in the method of the present invention is sphingomyelin.

More preferably, the at least one biomarker determined in the method of the present invention is a carbohydrate as shown in Table 2a, more preferably, Maltose, Maltotriose or Mannose.

More preferably, the at least one biomarker determined in the method of the present invention is Coenzyme Q10 or Coenzyme Q9.

In another preferred embodiment of the method of the present invention, the at least one biomarker is a biomarker of category 2 and the subject, more preferably, exhibits underlying pancreatic diseases such as pancreatitis, a risk factor for developing pancreatic cancer.

A metabolite as used herein refers to at least one molecule of a specific metabolite up to a plurality of molecules of the said specific metabolite. It is to be understood further that a group of metabolites means a plurality of chemically different molecules wherein for each metabolite at least one molecule up to a plurality of molecules may be present. A metabolite in accordance with the present invention encompasses all classes of organic or inorganic chemical compounds including those being comprised by biological material such as organisms. Preferably, the metabolite in accordance with the present invention is a small molecule compound. More preferably, in case a plurality of metabolites is envisaged, said plurality of metabolites representing a metabolome, i.e. the collection of metabolites being comprised by an organism, an organ, a tissue, a body fluid or a cell at a specific time and under specific conditions.

In addition to the specific biomarkers recited in the specification, other biomarkers may be, preferably, determined as well in the methods of the present invention. Such biomarkers may include peptide or polypeptide biomarkers or glycosides such as the CA19.9 antigen.

The term "sample" as used herein refers to samples from body fluids, preferably, blood, plasma, serum, saliva or urine, or samples derived, e.g., by biopsy, from cells, tissues or organs, in particular from the heart. More preferably, the sample is a blood, plasma or serum sample, most preferably, a plasma sample. Biological samples can be derived from a subject as specified elsewhere herein. Techniques for obtaining the aforementioned different types of biological samples are well known in the art. For example, blood samples may be obtained by blood taking while tissue or organ samples are to be obtained, e.g., by biopsy.

The aforementioned samples are, preferably, pre-treated before they are used for the method of the present invention. As described in more detail below, said pre-treatment may include treatments required to release or separate the compounds or to remove excessive material or waste. Suitable techniques comprise centrifugation, extraction, fractioning, ultrafiltration, protein precipitation followed by filtration and purification and/or enrichment of compounds. Moreover, other pre-treatments are carried out in order to provide the compounds in a form or concentration suitable for compound analysis. For example, if gas-chromatography coupled mass spectrometry is used in the method of the present invention, it will be required to derivatize the compounds prior to the said gas chromatography. Suitable and necessary pre-treatments depend on the means used for carrying out the method of the invention and are well known to the person skilled in the art. Pre-treated samples as described before are also comprised by the term "sample" as used in accordance with the present invention.

The term "subject" as used herein relates to animals and, preferably, to mammals. More preferably, the subject is a primate and, most preferably, a human. The subject, preferably, is suspected to suffer from pancreatic cancer, i.e. it may already show some or all of the symptoms associated with the disease. Preferably, the subject, however, is besides the aforementioned diseases and disorders apparently healthy. The said subject, preferably, is at increased risk of developing pancreatic cancer (Brand RE et al ,Gut. 2007;56:1460-9). More preferably, such a subject being at increased risk has one or more relatives suffering from pancreatic cancer, has a defined genetic predisposition for developing pancreatic cancer, including but not exclusive to Peutz-Jeghers Syndrome, has one or more relatives suffering from pancreatitis, and/or has a defined genetic predisposition for developing pancreatitis.

The term "determining the amount" as used herein refers to determining at least one characteristic feature of a biomarker to be determined by the method of the present invention in the sample. Characteristic features in accordance with the present invention are features which characterize the physical and/or chemical properties including biochemical properties of a biomarker. Such properties include, e.g., molecular weight, viscosity, density, electrical charge, spin, optical activity, colour, fluorescence, chemoluminescence, elementary composition, chemical structure, capability to react with other compounds, capability to elicit a response in a biological read out system (e.g., induction of a reporter gene) and the like. Values for said properties may serve as characteristic features and can be determined by techniques well known in the art. Moreover, the characteristic feature may be any feature which is derived from the values of the physical and/or chemical properties of a biomarker by standard operations, e.g., mathematical calculations such as multiplication, division or logarithmic calculus. Most preferably, the at least one characteristic feature allows the determination and/or chemical identification of the said at least one biomarker and its amount. Accordingly, the characteristic value, preferably, also comprises information relating to the abundance of the biomarker from which the characteristic value is derived. For example, a characteristic value of a biomarker may be a peak in a mass spectrum. Such a peak contains characteristic information of the biomarker, i.e. the m/z information, as well as an intensity value being related to the abundance of the said biomarker (i.e. its amount) in the sample.

As discussed before, each biomarker comprised by a sample may be, preferably, determined in accordance with the present invention quantitatively or semi-quantitatively. For quantitative determination, either the absolute or precise amount of the biomarker will be determined or the relative amount of the biomarker will be determined based on the value determined for the characteristic feature(s) referred to herein above. The relative amount may be determined in a case were the precise amount of a biomarker can or shall not be determined. In said case, it can be determined whether the amount in which the biomarker is present is enlarged or diminished with respect to a second sample comprising said biomarker in a second amount. In a preferred embodiment said second sample comprising said biomarker shall be a calculated reference as specified elsewhere herein. Quantitatively analysing a biomarker, thus, also includes what is sometimes referred to as semi-quantitative analysis of a biomarker.

Moreover, determining as used in the method of the present invention, preferably, includes using a compound separation step prior to the analysis step referred to before. Preferably, said compound separation step yields a time resolved separation of the metabolites comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be applied by the person skilled in the art without further ado. Most preferably, LC and/or GC are chromatographic techniques to be envisaged by the method of the present invention. Suitable devices for such determination of biomarkers are well known in the art. Preferably, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). Most preferably, LC-MS and/or GC-MS are used as described in detail below. Said techniques are disclosed in, e.g., Nissen 1995, Journal of Chromatography A, 703: 37-57, US 4,540,884 or US 5,397,894, the disclosure content of which is hereby incorporated by reference. As an alternative or in addition to mass spectrometry techniques, the following techniques may be used for compound determination: nuclear magnetic resonance (NMR), magnetic resonance imaging (MRI), Fourier transform infrared analysis (FT-IR), ultraviolet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado. The method of the present invention shall be, preferably, assisted by automation. For example, sample processing or pre-treatment can be automated by robotics. Data processing and comparison is, preferably, assisted by suitable computer programs and databases. Automation as described herein before allows using the method of the present invention in high-throughput approaches.

Moreover, the at least one biomarker can also be determined by a specific chemical or biological assay. Said assay shall comprise means which allow to specifically detect the at least one biomarker in the sample. Preferably, said means are capable of specifically recognizing the chemical structure of the biomarker or are capable of specifically identifying the biomarker based on its capability to react with other compounds or its capability to elicit a response in a biological read out system (e.g., induction of a reporter gene). Means which are capable of specifically recognizing the chemical structure of a biomarker are, preferably, antibodies or other proteins which specifically interact with chemical structures, such as receptors or enzymes. Specific antibodies, for instance, may be obtained using the biomarker as antigen by methods well known in the art. Antibodies as referred to herein include both polyclonal and monoclonal antibodies, as well as fragments thereof, such as Fv, Fab and $F(ab)_2$ fragments that are capable of binding the antigen or hapten. The present invention also includes humanized hybrid antibodies wherein amino acid sequences of a non-human donor antibody exhibiting a desired antigen-specificity are combined with sequences of a human acceptor antibody. Moreover, encompassed are single chain antibodies. The donor sequences will usually include at least the antigen-binding amino acid residues of the donor but may comprise other structurally and/or functionally relevant amino acid residues of the donor antibody as well. Such hybrids can be prepared by several methods well known in the art. Suitable proteins which are capable of specifically recognizing the biomarker are, preferably, enzymes which are involved in the metabolic conversion of the said biomarker. Said enzymes may either use the biomarker as a substrate or may convert a substrate into the biomarker. Moreover, said antibodies may be used as a basis to generate oligopeptides which specifically recognize the biomarker. These oligopeptides shall, for example, comprise the enzyme's binding domains or pockets for the said biomarker. Suitable antibody and/or enzyme based assays may be RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests. Moreover, the biomarker may also be determined based on its capability to react with other compounds, i.e. by a specific chemical reaction. Further, the biomarker may be determined in a sample due to its capability to elicit a response in a biological read out system. The biological response shall be detected as read out indicating the presence and/or the amount of the biomarker comprised by the sample. The biological response may be, e.g., the induction of gene expression or a phenotypic response of a cell or an organism. In a preferred embodiment the determination of the least one biomarker is a quantitative process, e.g., allowing also the determination of the amount of the at least one biomarker in the sample

As described above, said determining of the at least one biomarker can, preferably, comprise mass spectrometry (MS). Mass spectrometry as used herein encompasses all techniques which allow for the determination of the molecular weight (i.e. the mass) or a mass variable corresponding to a compound, i.e. a biomarker, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF or any combined approaches using the aforementioned techniques. How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, c) selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionisation process, whereby the quadrupole is filled with collision gas but no acceleration voltage is applied during the analysis. Details on said most preferred mass spectrometry to be used in accordance with the present invention can be found in WO 03/073464.

More preferably, said mass spectrometry is liquid chromatography (LC) MS and/or gas chromatography (GC) MS. Liquid chromatography as used herein refers to all techniques which allow for separation of compounds (i.e. metabolites) in liquid or supercritical phase. Liquid chromatography is characterized in that compounds in a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatised prior to gas chromatography. Suitable techniques for derivatisation are well known in the art. Preferably, derivatisation in accordance with the present invention relates to methoxymation and trimethylsilylation of, preferably, polar compounds and transmethylation, methoxymation and trimethylsilylation of, preferably, non-polar (i.e. lipophilic) compounds.

The term "reference" refers to values of characteristic features of each of the biomarker which can be correlated to a medical condition, i.e. the presence or absence of the disease, diseases status or an effect referred to herein. Preferably, a reference is a threshold value (e.g., an amount or ratio of amounts) for a biomarker whereby values found in a sample to be investigated which are higher than or essentially identical to the threshold are indicative for the presence of a medical condition while those being lower are indicative for the absence of the medical condition. It will be understood that also preferably, a reference may be a threshold value for a biomarker whereby values found in a sample to be investigated which are lower or identical than the threshold are indicative for the presence of a medical condition while those being higher are indicative for the absence of the medical condition.

In accordance with the aforementioned method of the present invention, a reference is, preferably, a reference obtained from a sample from a subject or group of subjects known to suffer from pancreatic cancer. In such a case, a value for the at least one biomarker found in the test sample being essentially identical is indicative for the presence of the disease. Moreover, the reference, also preferably, could be from a subject or group of subjects known not to suffer from pancreatic cancer, preferably, an apparently healthy subject. In such a case, a value for the at least one biomarker found in the test sample being altered with respect to the reference is indicative for the presence of the disease. The same applies mutatis mutandis for a calculated reference being, most preferably, the average or median for the relative or absolute value of the at least one biomarker in a population of individuals (comprising the subject to be investigated). The absolute or relative values of the at least one biomarker of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

The value for the at least one biomarker of the test sample and the reference values are essentially identical, if the values for the characteristic features and, in the case of quantitative determination, the intensity values are essentially identical. Essentially identical means that the difference between two values is, preferably, not significant and shall be characterized in that the values for the intensity are within at least the interval between $1^{st}$ and $99^{th}$ percentile, $5^{th}$ and $95^{th}$ percentile, $10^{th}$ and $90^{th}$ percentile, $20^{th}$ and $80^{th}$ percentile, $30^{th}$ and $70^{th}$ percentile, $40^{th}$ and $60^{th}$ percentile of the reference value, preferably, the $50^{th}$, $60^{th}$, $70^{th}$, $80^{th}$, $90^{th}$ or $95^{th}$ percentile of the reference value. Statistical test for determining whether two amounts are essentially identical are well known in the art and are also described elsewhere herein.

An observed difference for two values, on the other hand, shall be statistically significant. A difference in the relative or absolute value is, preferably, significant outside of the interval between $45^{th}$ and $55^{th}$ percentile, $40^{th}$ and $60^{th}$ percentile, $30^{th}$ and $70^{th}$ percentile, $20^{th}$ and $80^{th}$ percentile, $10^{th}$ and $90^{th}$ percentile, $5^{th}$ and $95^{th}$ percentile, $1^{st}$ and $99^{th}$ percentile of the reference value. Preferred changes and ratios of the medians are described in the accompanying Tables as well as in the Examples.

Preferably, the reference, i.e. values for at least one characteristic feature of the at least one biomarker or ratios thereof, will be stored in a suitable data storage medium such as a database and are, thus, also available for future assessments.

The term "comparing" refers to determining whether the determined value of a biomarker is essentially identical to a reference or differs there from. Preferably, a value for a biomarker is deemed to differ from a reference if the observed difference is statistically significant which can be determined by statistical techniques referred to elsewhere in this description. If the difference is not statistically significant, the biomarker value and the reference are essentially identical. Based on the comparison referred to above, a subject can be assessed to suffer from the disease, or not.

For the specific biomarkers referred to in this specification, preferred values for the changes in the relative amounts or ratios (i.e. the changes expressed as the ratios of the medians) are found in the Tables, below. Based on the ratios of the metabolites found in a subject suffering from pancreatic cancer and an apparently healthy control and the calculated t-values as shown in Tables 2a, 2b, 3a, 3b, below, it can be derived whether an increase or a decrease of a given biomarker of Tables 2a, 2b, 3a, 3b is indicative for the presence of pancreatic cancer. Negative t-values for a biomarker indicate that a decrease is indicative while positive t-values indicate that an increase of the biomarker is indicative for pancreatic cancer. It will be understood that the reference in said cases is derived from a subject or group of subjects known not to suffer from pancreatic cancer or is a calculated reference as defined elsewhere herein.

The comparison is, preferably, assisted by automation. For example, a suitable computer program comprising algorithms for the comparison of two different data sets (e.g., data sets comprising the values of the characteristic feature(s)) may be used. Such computer programs and algorithms are well known in the art. Notwithstanding the above, a comparison can also be carried out manually.

Advantageously, it has been found in the study underlying the present invention that the amounts of the specific biomarkers referred to above are indicators for pancreatic cancer. Accordingly, the at least one biomarker as specified above in a sample can, in principle, be used for assessing whether a subject suffers from pancreatic cancer, or not. This is particularly helpful for an efficient diagnosis of the disease as well as for improving of the pre-clinical and clinical management of pancreatic cancer as well as an efficient monitoring of patients. Moreover, the findings underlying the present invention will also facilitate the development of efficient drug-based therapies or other interventions against pancreatic cancer as set forth in detail below.

The definitions and explanations of the terms made above apply mutatis mutandis for the following embodiments of the present invention except specified otherwise herein below.

The present invention also relates to a method for identifying whether a subject is in need for a therapy of pancreatic cancer or a change of therapy comprising the steps of the methods of the present invention and the further step of identifying a subject in need if pancreatic cancer is diagnosed.

The phrase "in need for a therapy of pancreatic cancer" as used herein means that the disease in the subject is in a status where therapeutic intervention is necessary or beneficial in order to ameliorate or treat pancreatic cancer or the symptoms associated therewith. Accordingly, the findings of the studies underlying the present invention do not only allow diagnosing pancreatic cancer in a subject but also allow for identifying subjects which should be treated by a pancreatic cancer therapy or whose pancreatic cancer therapy needs adjustment. Once the subject has been identified, the method may further include a step of making recommendations for a therapy of pancreatic cancer.

A therapy of pancreatic cancer as used in accordance with the present invention, preferably, comprises surgery, radiotherapy or drug treatment. Preferred surgery-based therapies include resection of the pancreas or parts thereof such as pancreaticoduodenectomy, tail pancreatectomy. total or partial pancreatoctomy, palliative bridging procedures. Drug-based therapies, preferably, include the administration of one or more drugs with antitumour properties including but not exclusive to platinum derivatives, fluoropyrimidines, pyrimidine analogues, Gemcitabine, antimetabolites, alkylating agents, anthracyclines, plant alkaloids, topoisomerase inhibitors, targeted antibodies and tryosine kinase inhibitors.

The present invention further relates to a method for determining whether a therapy against pancreatic cancer is successful in a subject comprising the steps of the methods of the present invention and the further step of determining whether a therapy is successful if no pancreatic cancer is diagnosed.

It is to be understood that a pancreatic cancer therapy will be successful if pancreatic cancer or at least some symptoms thereof are treated or ameliorated compared to an untreated subject. Moreover, a therapy is also successful as meant herein if the disease progression can be prevented or at least slowed down compared to an untreated subject.

The present invention also relates to a device or system for diagnosing pancreas cancer in a sample of a subject comprising:

(a) an analyzing unit for the said sample of the subject comprising a detector for at least one biomarker of Tables 2a, 2b, 3a, 3b, said detector allowing for the determination of the amount of the said at least one biomarker in the sample; and operatively linked thereto,

(b) an evaluation unit comprising a data processing unit and a data base, said data base comprising a stored reference and said data processing unit being capable of (i) carrying out a comparison of the amount of the at least one biomarker determined by the analyzing unit and the stored reference and (ii) generating an output information based on which the diagnosis can be established.

A device as used herein shall comprise at least the aforementioned units. The units of the device are operatively linked to each other. How to link the means in an operating manner will depend on the type of units included into the device. For example, where the detector allows for automatic qualitative or quantitative determination of the biomarker, the data obtained by said automatically operating analyzing unit can be processed by, e.g., a computer program in order to facilitate the assessment in the evaluation unit. Preferably, the units are comprised by a single device in such a case. Said device may accordingly include an analyzing unit for the biomarker and a computer or data processing device as evaluation unit for processing the resulting data for the assessment and for stabling the output information. Preferred devices are those which can be applied without the particular knowledge of a specialized clinician, e.g., electronic devices which merely require loading with a sample. The output information of the device, preferably, is a numerical value which allows drawing conclusions on the presence or absence of pancreatic cancer and, thus, is an aid for diagnosis. More preferably, the output information is a preliminary diagnosis based on the aforementioned numerical value, i.e. a classifier which indicates whether the subject suffers from pancreatic cancer or not. Such a preliminary diagnosis may need the evaluation of further information which can be provided in the device of the invention by including an expert knowledge database system.

Alternatively, the units can be implemented into a system comprising several devices which are operatively linked to each other. Depending on the units to be used for the system of the present invention, said means may be functionally linked by connecting each mean with the other by means which allow data transport in between said means, e.g., glass fiber cables, and other cables for high throughput data transport. Nevertheless, wireless data transfer between the means is also envisaged by the present invention, e.g., via LAN (Wireless LAN, W-LAN). A preferred system comprises means for determining biomarkers. Means for determining biomarkers as used herein encompass means for separating biomarkers, such as chromatographic devices, and means for metabolite determination, such as mass spectrometry devices. Suitable devices have been described in detail above. Preferred means for compound separation to be used in the system of the present invention include chromatographic devices, more preferably devices for liquid chromatography, HPLC, and/or gas chromatography. Preferred devices for compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other. Most preferably, LC-MS and/or GC-MS are used in the system of the present invention as described in detail elsewhere in the specification. Further comprised shall be means for comparing and/or analyzing the results obtained from the means for determination of biomarkers. The means for comparing and/or analyzing the results may comprise at least one databases and an implemented computer program for comparison of the results. Preferred embodiments of the aforementioned systems and devices are also described in detail below.

Furthermore, the present invention relates to a data collection comprising characteristic values of at least one biomarker being indicative for a medical condition or effect as set forth above (i.e. diagnosing pancreatic cancer in a subject, identifying whether a subject is in need for a therapy of pancreatic cancer or determining whether a pancreatic cancer therapy is successful).

The term "data collection" refers to a collection of data which may be physically and/or logically grouped together. Accordingly, the data collection may be implemented in a single data storage medium or in physically separated data storage media being operatively linked to each other. Preferably, the data collection is implemented by means of a database. Thus, a database as used herein comprises the data collection on a suitable storage medium. Moreover, the database, preferably, further comprises a database management system. The database management system is, preferably, a network-based, hierarchical or object-oriented database management system. Furthermore, the database may be a federal or integrated database. More preferably, the database will be implemented as a distributed (federal) system, e.g. as a Client-Server-System. More preferably, the database is structured as to allow a search algorithm to compare a test data set with the data sets comprised by the data collection. Specifically, by using such an algorithm, the database can be searched for similar or identical data sets being indicative for a medical condition or effect as set forth above (e.g. a query search). Thus, if an identical or similar data set can be identified in the data collection, the test data set will be associated with the said medical condition or effect. Consequently, the information obtained from the data collection can be used, e.g., as a reference for the methods of the present invention described above. More preferably, the data collection comprises characteristic values of all biomarkers comprised by any one of the groups recited above.

In light of the foregoing, the present invention encompasses a data storage medium comprising the aforementioned data collection.

The term "data storage medium" as used herein encompasses data storage media which are based on single physical entities such as a CD, a CD-ROM, a hard disk, optical storage media, or a diskette. Moreover, the term further includes data storage media consisting of physically separated entities which are operatively linked to each other in a manner as to provide the aforementioned data collection, preferably, in a suitable way for a query search.

The present invention also relates to a system comprising:

(a) means for comparing characteristic values of the at least one biomarker of a sample operatively linked to
(b) a data storage medium as described above.

The term "system" as used herein relates to different means which are operatively linked to each other. Said means may be implemented in a single device or may be physically separated devices which are operatively linked to each other. The means for comparing characteristic values of biomarkers, preferably, based on an algorithm for comparison as mentioned before. The data storage medium, preferably, comprises the aforementioned data collection or database, wherein each of the stored data sets being indicative for a medical condition or effect referred to above. Thus, the system of the present invention allows identifying whether a test data set is comprised by the data collection stored in the data storage medium. Consequently, the methods of the present invention can be implemented by the system of the present invention.

In a preferred embodiment of the system, means for determining characteristic values of biomarkers of a sample are comprised. The term "means for determining characteristic values of biomarkers" preferably relates to the aforementioned devices for the determination of metabolites such as mass spectrometry devices, NMR devices or devices for carrying out chemical or biological assays for the biomarkers.

Moreover, the present invention relates to a diagnostic means comprising means for the determination of at least one biomarker selected from any one of the groups referred to above.

The term "diagnostic means", preferably, relates to a diagnostic device, system or biological or chemical assay as specified elsewhere in the description in detail.

The expression "means for the determination of at least one biomarker" refers to devices or agents which are capable of specifically recognizing the biomarker. Suitable devices may be spectrometric devices such as mass spectrometry, NMR devices or devices for carrying out chemical or biological assays for the biomarkers. Suitable agents may be compounds which specifically detect the biomarkers. Detection as used herein may be a two-step process, i.e. the compound may first bind specifically to the biomarker to be detected and subsequently generate a detectable signal, e.g., fluorescent signals, chemiluminescent signals, radioactive signals and the like. For the generation of the detectable signal further compounds may be required which are all comprised by the term "means for determination of the at least one biomarker". Compounds which specifically bind to the biomarker are described elsewhere in the specification in detail and include, preferably, enzymes, antibodies, ligands, receptors or other biological molecules or chemicals which specifically bind to the biomarkers.

Further, the present invention relates to a diagnostic composition comprising at least one biomarker selected from any one of the groups referred to above.

The at least one biomarker selected from any of the aforementioned groups will serve as a biomarker, i.e. an indicator molecule for a medical condition or effect in the subject as set for the elsewhere herein. Thus, the biomarker molecules itself may serve as diagnostic compositions, preferably, upon visualization or detection by the means referred to in herein. Thus, a diagnostic composition which indicates the presence of a biomarker according to the present invention may also comprise the said biomarker physically, e.g., a complex of an antibody and the biomarker to be detected may serve as the diagnostic composition. Accordingly, the diagnostic composition may further comprise means for detection of the metabolites as specified elsewhere in this description. Alternatively, if detection means such as MS or NMR based techniques are used, the molecular species which serves as an indicator for the risk condition will be the at least one biomarker comprised by the test sample to be investigated. Thus, the at least one biomarker referred to in accordance with the present invention shall serve itself as a diagnostic composition due to its identification as a biomarker.

In general, the present invention contemplates the use of at least one biomarker of Tables 2a, 2b, 3a, 3b in a sample of a subject for diagnosing pancreatic cancer.

In view of the above, the following embodiments are particularly envisaged:

1. A method for diagnosing pancreas cancer in a subject comprising the steps of:

(a) determining in a sample of a subject suspected to suffer from pancreas cancer the amount of at least one biomarker from Tables 2a, 2b, 3a, 3b and
(b) comparing the said amount of the at least one biomarker with a reference, whereby pancreas cancer is to be diagnosed.

2. The method of embodiment 1, wherein said reference is derived from a sample of a subject or group of subjects known not to suffer from pancreatic cancer or is a calculated reference.

3. The method of embodiment 1, wherein said reference is derived from a sample of a subject or group of subjects known to suffer from pancreatic cancer.

4. A method for identifying whether a subject is in need of a pancreas cancer therapy comprising the steps of the method of any one of embodiments 1 to 3 and the further step of identifying a subject in need of a pancreas cancer therapy if said subject is to be diagnosed to suffer from pancreas cancer.

5. A method for determining whether a therapy against pancreatic cancer is successful in a subject comprising the steps of the method of any one of embodiments 1 to 3 and the further step of determining whether a therapy is successful if no pancreatic cancer is diagnosed.

6. The method of embodiment 4 or 5, wherein said pancreas cancer therapy comprises surgery, radiotherapy or drug treatment.

7. The method of any one of embodiments 1 to 6, wherein said sample is a plasma, blood or serum sample.

8. The method of any one of embodiments 1 to 7, wherein said subject is a human.

9. The method of any one of embodiments 1 to 9, wherein said pancreas cancer is pancreas adenocarcinoma.

10. The method of any one of embodiments 1 to 9, wherein said at least one biomarker is a biomarker of category 2.

11. The method of embodiment 10, wherein said subject exhibits pancreatitis as an underlying pancreatic disease.

12. A device for diagnosing pancreas cancer in a sample of a subject comprising:

a) an analyzing unit for the said sample of the subject comprising a detector for at least one biomarker of Tables 2a, 2b, 3a, 3b, said detector allowing for the determination of the amount of the said at least one biomarker in the sample; and operatively linked thereto,
(b) an evaluation unit comprising a data processing unit and a data base, said data base comprising a stored reference and said data processing unit being capable of (i) carrying out a comparison of the amount of the at least one biomarker determined by the analyzing unit and the stored reference and (ii) generating an output information based on which the diagnosis can be established.

13. Use of at least one biomarker from Tables 2a, 2b, 3a, 3b in a sample of a subject suspected to suffer from pancreatic cancer for diagnosing pancreatic cancer.

All references cited herein are herewith incorporated by reference with respect to their disclosure content in general or with respect to the specific disclosure contents indicated above.

The invention will now be illustrated by the following Examples which are not intended to restrict or limit the scope of this invention.

**Examples**

The invention will now be illustrated by the following Examples which are not intended to restrict or limit the scope of this invention.

**Example 1**: Sample preparation and MS Analysis

Analysis of 38 pancreatic adenocarcinoma plasma samples, 20 plasma samples of patients with alcohol induced liver cirrhosis and 41 plasma samples from patients suffering from alcohol induced chronic pancreatitis revealed 107 possible biomarker candidates which where classified in 2 categories. The analysis of the plasma samples was carried out as follows:

Plasma samples were prepared and subjected to LC-MS/MS and GC-MS or XLC-MS/MS (hormones) analysis as described in the following:

The sample were prepared in the following way: Proteins were separated by precipitation from blood plasma. After addition of water and a mixture of ethanol and dichlormethan the remaining sample was fractioned into an aqueous, polar phase and an organic, lipophilic phase (lipid fraction).

For the transmethanolysis of the lipid extracts a mixture of 140 $\mu$l of chloroform, 37 $\mu$l of hydrochloric acid (37% by weight HCl in water), 320 $\mu$l of methanol and 20 $\mu$l of toluene was added to the evaporated extract. The vessel was sealed tightly and heated for 2 hours at 100°C, with shaking. The solution was subsequently evaporated to dryness. The residue was dried completely.

The methoximation of the carbonyl groups was carried out by reaction with methoxyamine hydrochloride (20 mg/ml in pyridine, 100 $\mu$l for 1.5 hours at 60°C) in a tightly sealed vessel. 20 $\mu$l of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 100 $\mu$l of N-methyl-N-(trimethylsilyl)-2,2,2-trifluoroacetamide (MSTFA) was carried out for 30 minutes at 60°C, again in the tightly sealed vessel. The final volume before injection into the GC was 220 $\mu$l.

For the polar phase the derivatization was performed in the following way: The methoximation of the carbonyl groups was carried out by reaction with methoxyamine hydrochloride (20 mg/ml in pyridine, 50 $\mu$l for 1.5 hours at 60°C) in a tightly sealed vessel. 10 $\mu$l of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 50 $\mu$l of N-methyl-N-(trimethylsilyl)-2,2,2-trifluoroacetamide (MSTFA) was carried out for 30 minutes at 60°C, again in the tightly sealed vessel. The final volume before injection into the GC was 110 $\mu$l.

The GC-MS systems consist of an Agilent 6890 GC coupled to an Agilent 5973 MSD. The autosamplers are CompiPal or GCPal from CTC.

For the analysis usual commercial capillary separation columns (30 m x 0,25 mm x 0,25 $\mu$m) with different poly-methyl-siloxane stationary phases containing 0 % up to 35% of aromatic moieties, depending on the analysed sample materials and fractions from the phase separation step, were used (for example: DB-1ms, HP-5ms, DB-XLB, DB-35ms, Agilent Technologies). Up to 1 $\mu$L of the final volume was injected splitless and the oven temperature program was started at 70 °C and ended at 340 °C with different heating rates depending on the sample material and fraction from the phase separation step in order to achieve a sufficient chromatographic separation and number of scans within each analyte peak. Furthermore RTL (Retention Time Locking, Agilent Technologies) was used for the analysis and usual GC-MS standard conditions, for example constant flow with nominal 1 to 1.7 ml/min. and helium as the mobile phase gas, ionisation was done by electron impact with 70 eV, scanning within a m/z range from 15 to 600 with scan rates from 2.5 to 3 scans/sec and standard tune conditions.

The HPLC-MS systems consisted of an Agilent 1100 LC system (Agilent Technologies, Wald-bronn, Germany) coupled with an API 4000 Mass spectrometer (Applied Biosystem/MDS SCI-EX, Toronto, Canada). HPLC analysis was performed on commercially available reversed phase separation columns with C18 stationary phases (for example: GROM ODS 7 pH, Thermo Betasil C18). Up to 10 $\mu$L of the final sample volume of evaporated and reconstituted polar and lipophilic phase was injected and separation was performed with gradient elution using methanol/water/formic acid or acetonitrile/water/formic acid gradients at a flowrate of 200 $\mu$L/min.

Mass spectrometry was carried out by electrospray ionisation in positive mode for the non-polar (lipid) fraction and negative mode for the polar fraction using multiple-reaction-monitoring-(MRM)-mode and fullscan from 100 - 1000 amu.

Analysis of complex lipids in plasma samples:

Total lipids were extracted from plasma by liquid/liquid extraction using chloroform/methanol.

The lipid extracts were subsequently fractionated by normal phase liquid chromatography (NPLC) into eleven different lipid groups according to Christie (Journal of Lipid Research (26), 1985, 507-512).

The lipid classes Free fatty acids (FFA), Diacylglycerides (DAG),Triacylglycerides (TAG), Phosphatidylinositols (PI), Phosphatidylethanolamines (PE), Phosphatidylcholines (PC), Lysophosphatidylcholines (LPC), Free sterols (FS), Phosphatidylserines (PS) were measured by GC. The fractions were analyzed by GC-MS after derivatization with TMSH (Trimethyl sulfonium hydroxide), yielding the fatty acid methyl esters (FAME) corresponding to the acyl moieties of the class-separated lipids. The concentrations of FAME from C14 to C24 were determined in each fraction.

The lipid classes Cholesteryesters (CE) and Sphingomyelins (SM) were analyzed by LC-MS/MS using electrospray ionization (ESI) and atmospheric pressure chemical ionization (APCI) with detection of specific multiple reaction monitoring (MRM) transitions for cholesterylesters and sphingoymelins, respectively.

Analysis of steroids and catecholamines in plasma samples:

Steroids and their metabolites were measured by online SPE-LC-MS (Solid phase extraction-LC-MS). Catecholamines and their metabolites were measured by online SPE-LC-MS as described by Yamada et al [21]. Yamada H, Yamahara A, Yasuda S, Abe M, Oguri K, Fukushima S, Ikeda-Wada S: Dansyl chloride derivatization of methamphetamine: a methode with advantages for screening and analysis of methamphetamine in urine. Journal of Analytical Toxicology, 26(1): 17-22 (2002)

Analysis of eicosanoids in plasma samples

Eicosanoids and related were measured out of plasma by offline- and online-SPE LC-MS/MS (Solid phase extraction-LC-MS/MS) (Masoodi M and Nicolaou A: Rapid Commun Mass Spectrom. 2006 ; 20(20): 3023-3029. Absolute quantification was performed by means of stable isotope-labelled standards.

**Example 2:** Data evaluation

Plasma samples were analyzed in randomized analytical sequence design with pooled samples (so called "Pool") generated from aliquots of each sample. The raw peak data were normalized to the median of pool per analytical sequence to account for process variability (so called "ratios"). Ratios were log10 transformed to approach a normal distribution of data. Statistical analysis was done by a simple linear model (ANOVA) with the following fixed effects: Disease, body mass index (BMI), age, storage time (storage) and recruitment site (site):

$$Disease + BMI + age + storage + site$$

From the results of the ANOVA model, two different approaches for identification of biomarkers for pancreatic cancer were applied. These different approaches resulted in different biomarker categories that are explained in Table 1 and the identified biomarkers are listed in Tables 2a, 2b, 3a, 3b, below.

**Table 1:** Identification strategies of biomarker candidates for pancreatic cancer

| | | |
|---|---|---|
| **Statistical parameters of ANOVA model** | Estimated fold change | Fold change ratio of pancreatic carcinoma relative to control, estimated from ANOVA on log10-transformed ratios with age, site, storage and gender as fixed effects |
| | t-value | t-value of ANOVA on log10-transformed ratios with age, site, storage and gender as fixed effects, t-values give relative change in units of standard deviation. Negative t-values indicate decreases, positive indicate increases |
| | p-value | p-value of ANOVA on log10-transformed ratios with age, site, storage and gender as fixed effects, calculated from t-value by taking degrees of freedom and one- or two-sided test into consideration |
| | | |

(continued)

| Potential biomarker candidate for pancreatic carcinoma category | 1 | Pankreatic carcinoma p-value <0.2 AND (Liver cirrhosis/Pancreatitis 1-2 levels of significance weaker OR liver cirrhosis/Pancreatitis p-value <0.2 with other direction); levels of significance being <0.05; <0.1; <0.2 |
|---|---|---|
| | 2 | Pancreatic carcinoma AND Pankreatitis p-value <0.2 AND with same direction, cirrhosis p-value >0.2 |

**Table 2a:** List of identified biomarkers category 1 for pancreatic cancer in comparison relative to controls

| Metabolite | Direction of change | ANOVA result of pancreatic carcinoma relative to control | | |
|---|---|---|---|---|
| | | Estimated fold change | p-value | t-value |
| Coenzyme Q9 | down | 0.35 | 0.0000 | -4.89 |
| Maltotriose | up | 151.32 | 0.0000 | 9.91 |
| Maltose | up | 30.42 | 0.0000 | 8.07 |
| Coenzyme Q10 | down | 0.67 | 0.0235 | -2.28 |
| 2-Hydroxybutyrate | up | 3.02 | 0.0000 | 5.53 |
| 5-Hydroxyeicosatetraenoic acid (C20:trans[6]cis[8, 11,14]4) (5-HETE) | down | 0.16 | 0.0000 | -5.37 |
| Proline | down | 0.55 | 0.0000 | -5.24 |
| 13-Hydroxyoctadecadienoic acid (13-HODE) (C18: cis[9]trans[11]2) | down | 0.28 | 0.0000 | -5.24 |
| Ornithine | down | 0.57 | 0.0000 | -4.77 |
| trans-4-Hydroxyproline | down | 0.49 | 0.0000 | -4.63 |
| Phosphatidylcholine (C18:0, C18:2) | up | 1.03 | 0.0000 | 4.41 |
| Threonine | down | 0.72 | 0.0009 | -3.37 |
| Histidine | down | 0.69 | 0.0009 | -3.37 |
| Glucuronic acid | down | 0.43 | 0.0010 | -3.34 |
| Phenylalanine | down | 0.72 | 0.0013 | -3.26 |
| Tyrosine | down | 0.70 | 0.0025 | -3.07 |
| Campesterol | down | 0.53 | 0.0027 | -3.04 |
| Phosphatidylcholine (C18:0, C18:1) | down | 0.94 | 0.0029 | -3.01 |
| Galactose, lipid fraction | down | 0.78 | 0.0038 | -2.93 |
| Citrulline | down | 0.71 | 0.0050 | -2.84 |
| beta-Sitosterol | down | 0.56 | 0.0056 | -2.80 |
| | | | | |
| Phosphatidylcholine (C18:2, C20:4) | up | 1.09 | 0.0101 | 2.60 |
| Glycerol, lipid fraction | down | 0.60 | 0.0102 | -2.59 |
| Sucrose | down | 0.42 | 0.0113 | -2.56 |
| Urea | down | 0.64 | 0.0120 | -2.54 |
| 2-Hydroxypalmitic acid (C16:0) | down | 0.78 | 0.0129 | -2.51 |

(continued)

| Metabolite | Direction of change | ANOVA result of pancreatic carcinoma relative to control | | |
|---|---|---|---|---|
| | | Estimated fold change | p-value | t-value |
| 1,5-Anhydrosorbitol | down | 0.56 | 0.0139 | -2.48 |
| Normetanephrine | down | 0.61 | 0.0177 | -2.39 |
| Hexadecanol | down | 0.81 | 0.0184 | -2.38 |
| Uric acid | down | 0.72 | 0.0292 | -2.20 |
| Ceramide (d18:1, C24:1) | down | 0.73 | 0.0377 | -2.09 |
| 11-Deoxycortisol | up | 2.34 | 0.0391 | 2.08 |
| Phosphatidylcholine (C16:0, C18:2) | up | 1.02 | 0.0398 | 2.07 |
| Oleic acid (C18:cis[9]1) | down | 0.75 | 0.0459 | -2.01 |
| Aspartate | down | 0.68 | 0.0494 | -1.98 |
| scyllo-Inositol | down | 0.66 | 0.0671 | -1.84 |
| TAG (C16:0, C16:1) | down | 0.79 | 0.0762 | -1.78 |
| Androstenedione | down | 0.70 | 0.0895 | -1.71 |
| Serotonin (5-HT) | up | 1.68 | 0.0908 | 1.70 |
| Glucose, lipid fraction | down | 0.80 | 0.1068 | -1.62 |
| 3,4-Dihydroxyphenylglycol (DOPEG) | up | 1.63 | 0.1234 | 1.55 |
| Creatine | down | 0.75 | 0.1259 | -1.54 |
| gamma-Tocopherol | down | 0.78 | 0.1399 | -1.48 |
| Choline plasmalogen (C18, C20:4) | down | 0.90 | 0.1452 | -1.46 |
| 11-Hydroxyeicosatetraenoic acid (C20:cis [5,8,12,14]4) | down | 0.44 | 0.1464 | -1.46 |
| Threonic acid | down | 0.80 | 0.1590 | -1.41 |
| Docosahexaenoic acid (C22:cis[4,7,10,13,16,19]6) | up | 1.28 | 0.1652 | 1.39 |
| Mannose | up | 1.37 | 0.1818 | 1.34 |

| Table 2b: List of identified biomarkers category 1 from lipid analysis which are altered between patients suffering from pancreatic cancer in comparison relative to controlsMetabolite | Direction of change | ANOVA result of pancreatic carcinoma relative to control | | |
|---|---|---|---|---|
| | | Estimated fold change | p-value | t-value |
| SM_Sphingomyelin (d18:0,C18:0) | up | 2.24 | 0.0001 | 4.04 |
| SM_Sphingomyelin (d18:1,C20:1) | up | 1.55 | 0.0008 | 3.43 |
| SM_Sphingomyelin (d18:1,C22:1) | up | 1.49 | 0.0011 | 3.32 |
| SM_Sphingomyelin (d18:1,C24:2) | up | 1.34 | 0.0066 | 2.75 |
| SM_Sphingomyelin (d18:1,C18:1) | up | 1.21 | 0.0972 | 1.67 |
| SM_Sphingomyelin (d18:1,C23:1) | up | 1.18 | 0.1822 | 1.34 |
| MAG_Oleic acid (C18:cis9]1) | down | 0.43 | 0.0000 | -4.76 |
| FFA_Linolenic acid (C18:cis[9,12,15]3) | up | 2.33 | 0.0000 | 4.51 |

(continued)

| Table 2b: List of identified biomarkers category 1 from lipid analysis which are altered between patients suffering from pancreatic cancer in comparison relative to controls**Metabolite** | Direction of change | ANOVA result of pancreatic carcinoma relative to control | | |
|---|---|---|---|---|
| | | Estimated fold change | p-value | t-value |
| MAG_Palmitic acid (C16:0) | down | 0.54 | 0.0007 | -3.45 |
| TAG_Stearic acid (C18:0) | down | 0.42 | 0.0019 | -3.16 |
| MAG_Stearic acid (C18:0) | down | 0.79 | 0.0046 | -2.88 |
| TAG_trans-Vaccenic acid | down | 0.48 | 0.0156 | -2.44 |
| TAG_Eicosenoic acid (C20:cis[11]1) | down | 0.58 | 0.0226 | -2.30 |
| TAG_Palmitic acid (C16:0) | down | 0.62 | 0.0371 | -2.10 |
| PC_Docosahexaenoic acid (C22:cis[4,7,10,13,16,19]6) | up | 1.43 | 0.0546 | 1.94 |
| TAG_Oleic acid (C18:cis[9]1) | down | 0.68 | 0.0599 | -1.90 |
| FFA_Myristic acid (C14:0) | up | 1.49 | 0.0615 | 1.88 |
| TAG_Elaidic acid (C18:trans[9]1) | down | 0.62 | 0.0834 | -1.74 |
| SV _ Triacylglycerols | down | 0.56 | 0.0945 | -1.68 |
| PS_Stearic acid (C18:0) | down | 0.55 | 0.0954 | -1.68 |
| PS_Palmitic acid (C16:0) | down | 0.54 | 0.0974 | -1.67 |

| Lipidomics abbreviations | FFA | Free fatty acids |
|---|---|---|
| | TAG | Triacylglycerols |
| | MAG | Monoacylglycerols |
| | PS | Phosphatidylserines |
| | PC | Phosphatidylcholines |
| | SM | Sphingomyelins |
| | SV | Sum value |

Abbreviation scheme for fatty acids:

C24:1: Fatty acid with 24 Carbon atoms and 1 double bond in the carbon skeleton.

Table 3a: List of identified biomarkers category 2 for pancreatic cancer in comparison relative to controls

| Metabolite | Direction of change | ANOVA result of pancreatic carcinoma relative to control | | |
|---|---|---|---|---|
| | | Estimated fold change | p-value | t-value |
| 4-Hydroxy-3-methoxymandelic acid | down | 0.48 | 0.0000 | -4.62 |
| 12-Hydroxyeicosatetraenoic acid (C20:cis [5,8,10,14]4) | up | 3.35 | 0.0005 | 3.54 |
| myo-Inositol-2-phosphate | up | 1.36 | 0.0211 | 2.33 |
| Phosphatidylcholine (C18:0, C22:6) | up | 1.19 | 0.0366 | 2.10 |
| Phosphatidylcholine No 02 | up | 1.09 | 0.0408 | 2.06 |

(continued)

| Metabolite | Direction of change | ANOVA result of pancreatic carcinoma relative to control | | |
|---|---|---|---|---|
| | | Estimated fold change | p-value | t-value |
| erythro-Dihydrosphingosine | up | 1.33 | 0.1144 | 1.59 |
| Phosphate (inorganic and from organic phosphates) | up | 1.20 | 0.1339 | 1.51 |

Information on Phosphatidylcholine No 02

| Phosphatidylcholine No 02 | Phosphatidylcholine No 02 belongs to the class of glycerophosphatidylcholines. It exhibits the following characteristic ionic species when detected with LC/MS, applying electro-spray ionization (ESI) mass spectrometry: mass-to-charge ratio (m/z) of the positively charged ionic species is 808.4 (+/- 0.5). |
|---|---|

Table 3b: List of identified biomarkers category 2 from lipid analysis which are altered between patients suffering from pancreatic cancer in comparison relative to controls

| Metabolite | Direction of change | ANOVA result of pancreatic carcinoma relative to control | | |
|---|---|---|---|---|
| | | Estimated fold change | p-value | t-value |
| SM_Sphingomyelin (d18:1,C24:1) | up | 1.22 | 0.0699 | 1.83 |
| SM_Sphingomyelin (d18:1,C18:0) | up | 1.16 | 0.1447 | 1.47 |
| FFA_Elaidic acid (C18:trans[9]1) | up | 1.98 | 0.0002 | 3.88 |
| FFA_Arachidonic acid (C20:cis [5,8,11,14]4) | up | 1.51 | 0.0145 | 2.47 |
| PC_7Z, 10Z, 13Z, 16Z, 19Z-Docosapentaenoic acid | up | 1.37 | 0.0719 | 1.81 |
| PC_cis-Vaccenic acid | up | 1.25 | 0.0970 | 1.67 |
| PC_7Z, 10Z, 13Z, 16Z-Docosatetraenoic acid | up | 1.26 | 0.1062 | 1.62 |
| FFA_Stearic acid (C18:0) | up | 1.19 | 0.1732 | 1.37 |
| | | | | |
| **Lipidomics abbreviations** | FFA | Free fatty acids | | |
| | PC | Phosphatidylcholines | | |
| | SM | Sphingomyelins | | |

## Claims

1.  A method for diagnosing pancreas cancer in a subject comprising the steps of:

(a) determining in a sample of a subject suspected to suffer from pancreas cancer the amount of at least one biomarker from Tables 2a, 2b, 3a, 3b and
(b) comparing the said amount of the at least one biomarker with a reference, whereby pancreas cancer is to be diagnosed,

wherein said at least one biomarker comprises proline.

2. The method of claim 1, wherein said reference is derived from a sample of a subject or group of subjects known not to suffer from pancreatic cancer or is a calculated reference.

3. The method of claim 1, wherein said reference is derived from a sample of a subject or group of subjects known to suffer from pancreatic cancer.

4. A method for identifying whether a subject is in need of a pancreas cancer therapy comprising the steps of the method of any one of claims 1 to 3 and the further step of identifying a subject in need of a pancreas cancer therapy if said subject is to be diagnosed to suffer from pancreas cancer.

5. A method for determining whether a therapy against pancreatic cancer is successful in a subject comprising the steps of the method of any one of claims 1 to 3 and the further step of determining whether a therapy is successful if no pancreatic cancer is diagnosed.

6. The method of claim 4 or 5, wherein said pancreas cancer therapy comprises surgery, radiotherapy or drug treatment.

7. The method of any one of claims 1 to 6, wherein said sample is a plasma, blood or serum sample.

8. The method of any one of claims 1 to 7, wherein said subject is a human.

9. The method of any one of claims 1 to 9, wherein said pancreas cancer is pancreas adenocarcinoma.

10. The method of any one of claims 1 to 9, wherein said at least one biomarker is a biomarker of category 1.

11. The method of claim 10, wherein said subject exhibits pancreatitis as an underlying pancreatic disease.

12. A device for diagnosing pancreas cancer in a sample of a subject comprising:

b) an analyzing unit for the said sample of the subject comprising a detector for at least one biomarker of Tables 2a, 2b, 3a, 3b, said detector allowing for the determination of the amount of the said at least one biomarker in the sample; and operatively linked thereto,
(b) an evaluation unit comprising a data processing unit and a data base, said data base comprising a stored reference and said data processing unit being capable of (i) carrying out a comparison of the amount of the at least one biomarker determined by the analyzing unit and the stored reference and (ii) generating an output information based on which the diagnosis can be established,

wherein said at least one biomarker comprises proline.

13. Use of at least one biomarker from Tables 2a, 2b, 3a, 3b in a sample of a subject suspected to suffer from pancreatic cancer for diagnosing pancreatic cancer, wherein said at least one biomarker comprises proline.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4540884 A
- US 5397894 A
- WO 03073464 A

### Non-patent literature cited in the description

- **EVERHART.** *Gastroenterology,* 2009, vol. 136, 1134-11449
- **DEWITT.** *Gastroenterol Hepatol,* 2006, vol. 4, 717-25
- **FRY.** *Langenbecks Arch Surg.,* 2008, vol. 393, 883-90
- **HRUBAN.** *Am J Surg Path,* 2004, vol. 28, 977-987
- **LOWENFELS.** *N Engl J Med,* 1993, vol. 328, 1433-1437
- **HOWES.** *Clinical Gastroenterology and Hepatology,* 2004, vol. 2, 252-261
- **LOWENFELS.** *JAMA,* 2001, vol. 286, 169-170
- **LOWENFELS.** *J Natl Cancer Inst,* 1997, vol. 89, 442-44656
- **SCHRADER.** *Panceas,* 2009, vol. 38, 416-421
- **MODRAK.** *Mol Cancer Res,* 2009, vol. 7, 890-896
- **DOWDY ; WEARDEN.** Statistics for Research. John Wiley & Sons, 1983
- **BRAND RE et al.** *Gut.,* 2007, vol. 56, 1460-9
- **NISSEN.** *Journal of Chromatography A,* 1995, vol. 703, 37-57
- **CHRISTIE.** *Journal of Lipid Research,* 1985, vol. 26, 507-512
- **YAMADA H ; YAMAHARA A ; YASUDA S ; ABE M ; OGURI K ; FUKUSHIMA S ; IKEDA-WADA S.** Dansyl chloride derivatization of methamphetamine: a methode with advantages for screening and analysis of methamphetamine in urine. *Journal of Analytical Toxicology,* 2002, vol. 26 (1), 17-22
- **MASOODI M ; NICOLAOU A.** *Rapid Commun Mass Spectrom,* 2006, vol. 20 (20), 3023-3029